# FASCICULE DE BREVET EUROPEEN

(11) **EP 4 374 884 B1**
(45) Date de publication et mention de la délivrance du brevet: **17.12.2025**
(21) Numéro de dépôt: 23211974.3
(22) Date de dépôt: 24.11.2023
(51) Int. Cl.: A61L 2/10, B65B 55/08, C12H 1/16

(54) **DISPOSITIF DE DÉCONTAMINATION DE CONTENANT POUR LA FABRICATION DE VIN OU SIMILAIRE ET PROCÉDÉ DE MISE EN OEUVRE DE CE DISPOSITIF**
VORRICHTUNG ZUR DEKONTAMINATION VON BEHÄLTERN ZUR WEINHERSTELLUNG ODER DERGLEICHEN UND VERFAHREN ZUR VERWENDUNG DIESER VORRICHTUNG
DEVICE FOR DECONTAMINATING A CONTAINER FOR WINE MAKING OR THE LIKE AND METHOD FOR IMPLEMENTING SAID DEVICE

(30) Priorité: 25.11.2022 FR 2212316; 15.03.2023 FR 2302412
(43) Date de publication de la demande: 29.05.2024
(73) Titulaire: Bacchustorm, 30390 Domazan (FR)
(72) Inventeur: Puisnel, Christophe, 30400 Villeneuve-les-Avignon (FR); Franc, Janyce, 30400 Villeneuve-les-Avignon (FR)
(74) Mandataire: Rhein, Alain

(56) Documents cités:
- ES-U- 1 265 944
- US-A1- 2018 207 303
- US-A1- 2020 230 273

## Description

La présente invention concerne le domaine de la décontamination au moyen de contenant pour la fabrication de vin ou similaire, tel que des barriques, des cuves en inox ou béton, des foudres, des amphores, etc. ceci au moyen de rayonnement ultraviolet, notamment de type lumière pulsée.

Dans l'état de la technique connu, plusieurs méthodes de décontamination des barriques de vin sont utilisées. Ces derrières emploient, par exemple, de la vapeur, de l'eau chaude sous pression, des ultrasons, de l'eau ozonée, un mélange de soude et de permanganate et/ou une mèche de soufre. Cette dernière méthode est la plus répandue pour décontaminer les barriques de vin. Cependant, l'utilisation de soufre n'est pas sans risques pour la santé humaine, c'est pourquoi il existe un besoin de trouver des solutions alternatives à cette méthode.

La décontamination par rayonnement ultraviolet, s'avère être une solution plus efficace, plus écologique et plus saine que les méthodes de décontamination précitées.

Plus précisément, il est connu d'utiliser des lampes à rayonnement ultraviolet ou lampe UV-C que l'on vient insérer dans le contenant à décontaminer. C'est par exemple le cas du document US 2018/207303 qui décrit un dispositif UV pour la stérilisation d'un récipient de vin, comprenant une lampe UV, un capteur de luminosité, ainsi qu'un écran de protection et un support de montage pour fixer l'appareil. Toutefois, ce document ne divulgue pas de fourreau coulissant, ni un mécanisme à ressort de rappel. 1

Le document ES1265944 U divulgue un dispositif de décontamination de contenant pour la fabrication de vin ou d'autres boissons alcoolisées au moyen de rayonnement ultraviolet. Ledit dispositif comprend un corps principal comportant une lampe à rayonnement ultraviolet ou lampe UV-C, un fourreau rétractable pouvant coulisser le long dudit corps principal entre : une position de repos dans laquelle ladite lampe UV- C est entièrement insérée à l'intérieure dudit corps principal et une position d'utilisation dans laquelle ladite lampe UV-C s'étend en dehors dudit corps principal à une seconde extrémité inférieure de ce dernier. Ledit dispositif comporte également des moyens d'actionnement permettant d'assurer le coulissement dudit fourreau entre les deux positions. Un ressort permet de maintenir ladite lampe UV-C en position de repos tant que les moyens d'actionnement ne sont pas actionnés. Un moyen de maintien est également décrit afin de compenser l'action dudit ressort et de maintenir ladite lampe UV-C dans ledit contenant dans sa position d'utilisation.

Ce dispositif nécessite l'utilisation d'une grille de support muni de pieds et venant en appui contre ledit contenant. Ladite lampe UV-C est alors maintenue en position sur ladite grille pour être ensuite insérée, à travers ladite grille, dans ledit contenant. Cette configuration a pour inconvénient de rendre l'installation de ladite lampe UV-C dans ledit contenant longue et fastidieuse.

Le document US2020/0230273 A1 pallie cet inconvénient en proposant un support sous forme d'une embase fixée directement sur un fourreau rétractable. Plus précisément, ce document décrit un dispositif portable de décontamination à rayonnement ultraviolet d'un contenant pour la fabrication de vin. Ledit dispositif comprend : un corps principal, une lampe à rayonnement ultraviolet ou lampe UV-C fixée à une seconde extrémité inférieure dudit corps principal, un fourreau rétractable monté coulissant sur ledit corps principal entre une position d'utilisation et une position de repos, un moyen de préhension dudit dispositif. Ledit moyen de préhension est fixé à une première extrémité supérieure dudit corps principal. Lorsque ladite lampe UV-C est insérée dans ledit contenant, ledit fourreau rétractable vient en appui, par l'intermédiaire de ladite embase, contre ledit contenant et coulisse le long dudit corps principal. Ledit fourreau rétractable se retrouve alors dans une position d'utilisation dans laquelle ladite lampe UV-C s'étend en dehors dudit fourreau rétractable à une deuxième extrémité inférieure de ce dernier. Lorsque ladite lampe UV-C est retirée dudit contenant, un ressort exerce une force de rappel de sorte à permettre audit fourreau rétractable de coulisser le long dudit corps principal afin de recouvrir ladite lampe UV-C, ledit fourreau rétractable se retrouve alors dans une position de repos.

Bien que ce dispositif facilite l'installation de la lampe UV-C, il présente cependant des inconvénients lors du retrait de ladite lampe UV-C par un opérateur. En effet, face à la force de rappel exercée par ledit ressort lors du retrait de ladite lampe UV-C, l'opérateur doit alors exercer une force de résistance afin d'éviter un dégagement trop rapide du dispositif pouvant endommager ladite lampe UV-C. De plus, le moyen de préhension dudit dispositif est constitué d'un capuchon tubulaire et ne permet pas une bonne prise en main par l'opérateur, rendant ainsi l'utilisation de ladite lampe UV-C difficile et pouvant endommager cette dernière en cas de mauvaise prise en main. Il existe donc un besoin de faciliter l'installation, le retrait et la maniabilité dudit dispositif.

Par ailleurs, le fourreau rétractable décrit par le document US2020/0230273 est transparent ainsi, si ladite lampe UV-C est allumée alors que cette dernière n'est pas encore insérée dans ledit contenant, l'opérateur court un risque d'exposition à la lumière. Or, l'exposition à la lumière ultraviolette peut être très nocive pour la santé.

Bien que le document ES1265944 U décrive un fourreau rétractable opaque, permettant de limiter l'exposition à la lumière, il est cependant nécessaire, avant l'insertion de ladite lampe UV-C dans ledit contenant, d'allumer cette dernière et de vérifier si cette dernière est bien allumée. L'opérateur est alors exposé au rayonnement ultraviolet de ladite lampe UV-C. Il existe donc un besoin d'améliorer la sécurité des opérateurs manipulant un tel dispositif.

La présente invention a pour but de remédier à ces divers inconvénients en proposant un dispositif de décontamination de contenant, pour la fabrication de vin ou similaire, d'installation et de manipulation aisée et assurant la sécurité des opérateurs. A ce propos, l'objectif de l'invention consiste à éviter tout risque d'irradiation aux rayonnement UV de l'opérateur que ce soit en raison d'une défaillance ou une erreur de manipulation du dispositif. En particulier, le fonctionnement de la lampe ne doit être rendu possible qu'après s'être assuré qu'il n'y a pas de risque de fuite de rayonnement UV du contenant dans laquelle est introduite la lampe.

C'est dans le cadre d'une première démarche inventive qu'il a été imaginé d'équiper le dispositif d'un capteur d'obscurité pour répondre à ce problème. Ce capteur peut agir tant en début de phase opératoire qu'à la fin et ainsi détecter toute rupture de cette obscurité pour en même temps commander la coupure de fonctionnement de la lampe.

Dans une seconde démarche inventive, il a encore été imaginer d'équiper le dispositif de décontamination de moyens de détection de position du fourreau rétractable. Le capteur de position est configuré pour détecter la position de fin de course du fourreau rétractable en position d'utilisation. Le dispositif d'allumage de la lampe UV-C ne peut être activé tant que les moyens de détection de position n'ont pas détecté que le fourreau rétractable est arrivé en fin de course.

Cette configuration permet de s'assurer que le dispositif de décontamination est correctement inséré dans le contenant à décontaminer, plus particulièrement, que la lampe UV-C est entièrement insérée à l'intérieur du contenant et n'est plus visible pour un opérateur, afin de lui éviter une exposition au rayonnement ultraviolet.

Ainsi, l'allumage de la lampe UV-C ne peut être activé que lorsqu'elle est entièrement placée dans l'obscurité et que le fourreau rétractable est arrivé en fin de course.

A cet effet, la présente invention concerne un dispositif de décontamination conforme à la revendication 1.Préférentiellement, ces moyens de détection de position peuvent comprendre un capteur de position du fourreau rétractable et une butée de fin de course ou encore un contacteur mécanique à levier. Le capteur de position est configuré pour détecter la position de fin de course du fourreau rétractable en position d'utilisation. Le dispositif d'allumage de la lampe UV-C ne peut être activé tant que les moyens de détection de position n'ont pas détecté que le fourreau rétractable est arrivé en fin de course.

Selon une autre caractéristique additionnelle, le dispositif comporte des moyens de maintien de ce dernier dans le contenant à décontaminer lors de la position d'utilisation du dispositif.

Selon une autre caractéristique additionnelle, les moyens de maintien sont situés sur une première extrémité supérieure de la lampe UV-C.

Selon une autre caractéristique additionnelle, les moyens de maintien comportent au moins trois pattes de maintien, préférentiellement quatre pattes de maintiens. Les dites pattes de maintien sont de préférence métalliques.

Selon une autre caractéristique additionnelle, les pattes de maintien sont configurées pour se déployer après l'insertion du dispositif dans une ouverture que comporte le contenant à décontaminer.

Ainsi, une fois le dispositif inséré dans ce contenant à décontaminer, il est maintenu en position et bloqué contre le contenant à décontaminer par l'intermédiaire de l'embase et des pattes de maintien.

Selon une autre caractéristique additionnelle, les moyens de préhension du dispositif de décontamination prennent préférentiellement la forme d'une poignée. Cette caractéristique permet de faciliter la préhension du dispositif de décontamination par un opérateur.

Selon une autre caractéristique additionnelle, le dispositif comprend des moyens de positionnement du dispositif en position verticale ou horizontale. De manière avantageuse les moyens de positionnement sont réalisés sous forme de crochets.

Selon une autre caractéristique additionnelle, le fourreau rétractable comporte des moyens d'évacuation d'air qui permettent d'évacuer la chaleur dissipée par la lampe UV-C lors de son utilisation, préférentiellement formés par des orifices d'évacuation.

Selon une autre caractéristique additionnelle, le dispositif comprend des moyens de mesure du rayonnement émis par la lampe UV-C, préférentiellement constitués d'une photodiode. Cette caractéristique permet de détecter le niveau d'usure de la lampe UV- C.

Selon une autre caractéristique de l'invention, le dispositif de décontamination comporte des moyens de raccordement d'alimentation à un poste d'alimentation et de pilotage. Celui-ci comprend avantageusement un chariot, muni d'une station d'accueil dudit dispositif de décontamination. Ce poste d'alimentation et de pilotage est également équipé, préférentiellement, de moyens de déplacement et de moyens de manipulation, permettant à l'opérateur de se déplacer aisément auprès des contenants en facilitant ainsi l'utilisation du dispositif de décontamination.

Le poste d'alimentation et de pilotage comprend également un pupitre de commande, avantageusement équipé d'un écran de pilotage évitant à l'opérateur d'intervenir directement sur le dispositif de décontamination lors de son fonctionnement.

Ce poste d'alimentation et de pilotage peut encore être pourvu de moyens de connexion auxiliaire pour le raccordement sur ce poste d'autres types d'outils de décontamination, par exemple.

Le poste d'alimentation et de pilotage comporte des moyens de raccordement à un réseau d'alimentation électrique.

Dans une variante envisageable, il peut, le cas échéant, accueillir également une alimentation autonome, telle qu'au moins une batterie rechargeable.

L'invention concerne encore un procédé de mise en œuvre d'un dispositif de décontamination conforme à l'invention pour la décontamination d'un contenant alimentaire pour la fabrication de vin ou d'autres boissons alcoolisées, par rayonnement ultraviolet, notamment de type lumière pulsée, ce contenant comportant au moins une ouverture.

Le procédé se caractérise en ce qu'il comporte :
- une étape d'application de l'embase à la seconde extrémité inférieure du fourreau rétractable au-dessus de l'ouverture du contenant ;
- une étape d'engagement de la lampe UV-C à l'intérieur du contenant au travers de ladite ouverture en repoussant le fourreau rétractable depuis sa position de repos dans sa position d'utilisation, contre l'action de moyens de rappel élastique ;
- une étape d'accrochage du dispositif de décontamination sur le contenant ;
- une étape de vérification comprenant la vérification d'au moins deux conditions de sécurité parmi les suivantes :
   ∘ le moyen de détection d'obscurité détecte que la lampe UV-C est placée dans l'obscurité ;
   ∘ le capteur de position du fourreau rétractable détecte la fin de course de ce dernier qui vient en butée contre le contenant ;
- une étape de transmission comprenant la transmission au poste d'alimentation et de pilotage et/ou au dispositif d'allumage de la lampe UV-C d'informations de vérification des conditions de sécurité ;
- une étape de validation dans laquelle :
   ∘ si au moins une des deux conditions de sécurité n'est pas vérifiée alors une alerte est émise audit poste d'alimentation et de pilotage et/ou au dispositif d'allumage de ladite lampe UV-C afin de contrôle et/ou réitération des étapes précédentes ;
   ∘ si toutes les conditions de sécurité sont vérifiées, le dispositif d'allumage de la lampe UV-C 4 peut alors être activé.

D'autres particularités et avantages apparaitront dans la description détaillée, qui suit, d'un exemple de réalisation, non limitatif, de l'invention illustré par les figures 1 à 5 annexées et dans lesquelles :
[Fig.1] représente une vue schématique d'un dispositif de décontamination d'un contenant conforme à l'invention lorsque le fourreau rétractable est en position de repos ;
[Fig.2] représente une vue schématique d'une coupe longitudinale du dispositif conforme à l'invention lorsque le fourreau rétractable est en position de repos ;
[Fig. 3] représente une vue schématique d'un dispositif de décontamination d'un contenant selon l'invention lorsque le fourreau rétractable est en position d'utilisation ;
[Fig.4] représente une vue schématique du poste d'alimentation et de pilotage auquel est raccordé le dispositif de décontamination selon l'invention ;
[Fig.5] représente une vue schématique de l'utilisation d'un dispositif de décontamination selon l'invention.

La présente invention se rapporte à un dispositif de décontamination 1 de contenant 100 pour la fabrication de vin ou d'autres boissons alcoolisées par rayonnement ultraviolet et plus particulièrement par lumière pulsée.

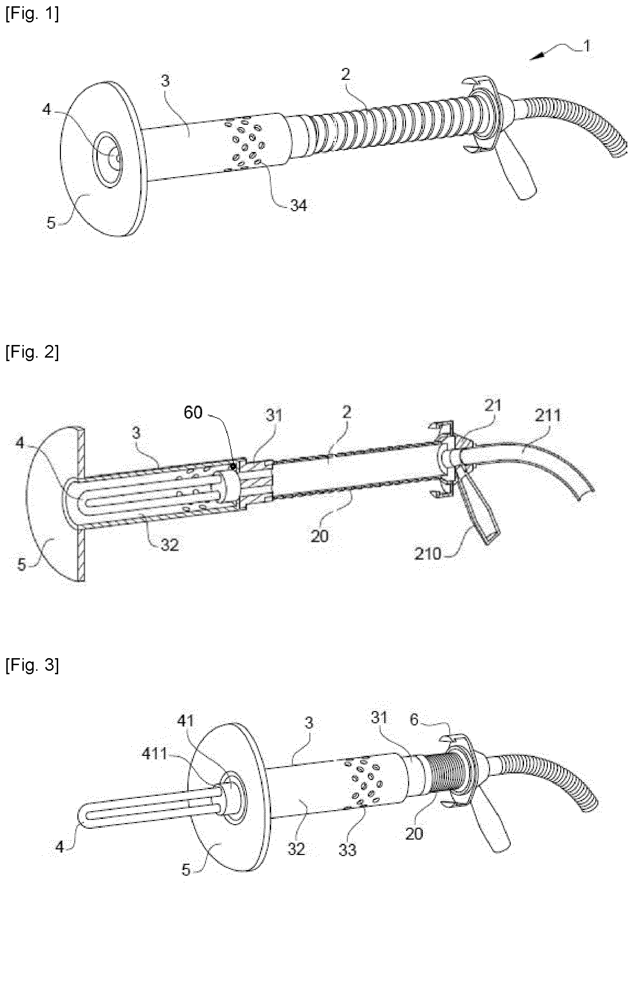

Comme illustré dans les figures 1 et 2, le dispositif de décontamination 1 comprend un corps principal 2, préférentiellement cylindrique. Ce corps principal 2 comporte à une première extrémité supérieure des moyens de préhension empruntant avantageusement la forme d'une poignée 210. Le dispositif de décontamination est également muni d'une lampe UV-C 4, préférentiellement une lampe à lumière pulsée. Le corps principal 2 est prolongé par la lampe UV-C 4 qui s'étend sensiblement selon l'axe longitudinal du corps principal 2. Plus particulièrement, la lampe UV-C 4 est fixée sur une seconde extrémité inférieure du corps principal 2.

Le dispositif de décontamination 1 comporte également un fourreau rétractable 3 comprenant une première extrémité supérieure et une seconde extrémité inférieure. Le fourreau rétractable 3 est muni d'une embase 5. Cette embase 5 est située sur la seconde extrémité inférieure du fourreau rétractable 3.

Dans ces figures 1 et 2, le fourreau rétractable 3 est positionné dans une position de repos dans laquelle la lampe UV-C 4 est entièrement insérée à l'intérieur du fourreau rétractable 3. Ce fourreau rétractable 3 est conçu en un matériau opaque aux rayonnements UV-C au moins dans sa portion s'étendant autour de la lampe UV-C 4 dans cette position dans laquelle celle-ci est entièrement insérée à l'intérieur de ce fourreau 3.

La figure 3 illustre le fourreau rétractable 3 dans une position d'utilisation dans laquelle la lampe UV-C 4 s'étend en dehors dudit fourreau rétractable 3 à une seconde extrémité inférieure de ce dernier.

Plus particulièrement, le fourreau rétractable 3 est monté coulissant sur le corps principal 2 entre une position de repos dans laquelle ladite lampe UV-C 4 est entièrement insérée dans ledit fourreau rétractable 3 et une position d'utilisation dans laquelle ladite lampe UV-C 4 s'étend en dehors dudit fourreau rétractable 3 à une seconde extrémité inférieure de ce dernier.

Le fourreau rétractable 3 comprend une portion principale 32 et une portion de liaison 31. La portion principale 32, préférentiellement en un matériau opaque aux rayonnements UV-C, s'étend depuis la seconde extrémité inférieure du fourreau rétractable 3 jusqu'à la portion de liaison 31. La portion de liaison 31 est située dans la continuité de la portion principale 32 et s'étend depuis la portion principale 32 jusqu'à la première extrémité supérieure dudit fourreau rétractable 3. Le diamètre de la portion de liaison 31 est inférieur au diamètre de la portion principale 32. Le fourreau rétractable 3 coulisse sur le corps principal 2 par l'intermédiaire de la portion de liaison 31.

Le dispositif de décontamination 1 comprend également des moyens de rappel élastique, préférentiellement constitués d'un ressort 20. Le ressort 20 est fixé au niveau de la première extrémité supérieure du fourreau rétractable 3. Plus particulièrement, le ressort 20 est fixé à la portion de liaison 31 et est positionné autour du corps principal 2, préférentiellement cylindrique. Lors du retrait du dispositif de décontamination 1 hors du contenant, les moyens de rappel élastique permettent au fourreau rétractable 3 de retrouver sa position de repos.

De plus, le dispositif de décontamination 1 comporte des moyens d'actionnement configurés pour permettre, lors du retrait dudit dispositif 1 hors dudit contenant 100, un retour progressif dudit fourreau rétractable 3 depuis ladite position d'utilisation vers ladite position de repos, ceci en assurant tout du long le maintien en applique de l'embase 5 au-dessus de l'ouverture 108 du contenant 100. Ceci permet d'éviter tout risque d'exposition aux rayonnement ultraviolet si, comme cela sera expliqué plus en détail ci-après, la lampe restait en fonctionnement malgré les sécurités particulières prévues par l'invention.

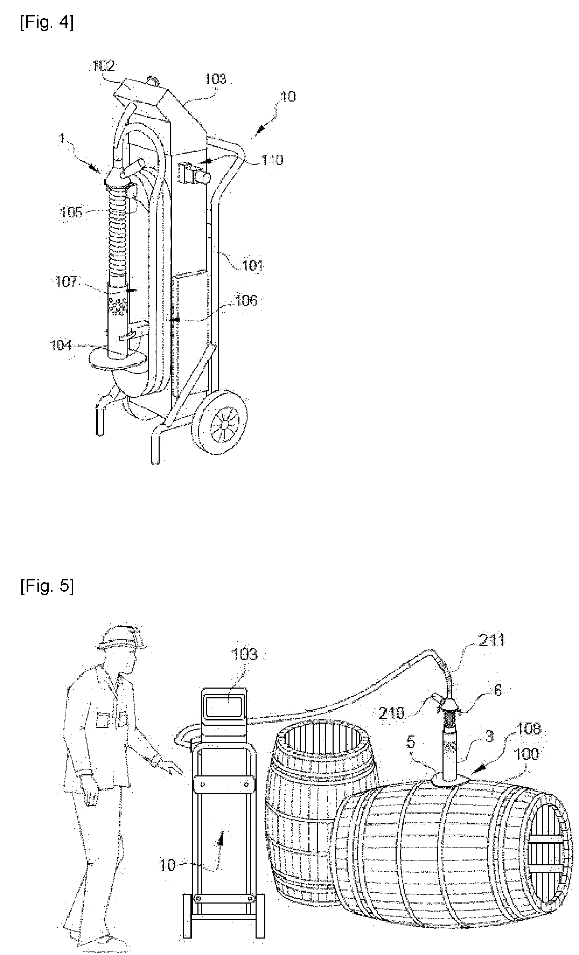

La figure 4 illustre un poste d'alimentation et de pilotage 10 auquel est raccordé le dispositif de décontamination 1 par des moyens de raccordement d'alimentation 211.

A noter, que ces moyens de raccordement d'alimentation 211 sont avantageusement branchés de manière apte à être déconnectés au poste d'alimentation et de pilotage 10. En particulier, lesdits moyens de raccordement d'alimentation 211 peuvent être équipés, à leur extrémité opposée au dispositif de décontamination 1, d'une prise de branchement, préférentiellement de type mâle, conçue pour coopérer avec une prise de raccordement préférentiellement de type femelle équipant le poste d'alimentation et de pilotage 10. Cette configuration permet le raccordement éventuel de ce poste d'alimentation et de pilotage 10 d'autres types d'outil de décontamination par rayonnement UV-C.

Le poste d'alimentation et de pilotage 10 comprend un chariot 101 muni de moyens de déplacement tel que, par exemple, des roues. Le chariot 101 comprend également des moyens de prise en main, notamment sous forme de poignées. Le poste d'alimentation et de pilotage 10 comporte préférentiellement un pupitre de commande 102 avantageusement pourvu d'un écran de pilotage 103.

Ce poste d'alimentation et de pilotage 10 comporte des moyens, non représentés, de raccordement à une réseau d'alimentation électrique d'un bâtiment. Selon une variante possible, il peut également être muni d'une alimentation autonome, comportant au moins une batterie rechargeable.

Comme représenté, ce poste d'alimentation et de pilotage 10 comprend selon l'invention une station d'accueil 107 du dispositif de décontamination 1 sous forme de moyens d'accrochage et/ou de fixation 104, 105, voire encore sous forme de moyens d'enroulement 106 des moyens de raccordement d'alimentation 211. De préférence, la station d'accueil 107 du dispositif de décontamination 1 est ménagée sur l'un des côtés verticaux du poste d'alimentation et de pilotage 10.

Ce poste d'alimentation et de pilotage 10 peut encore être pourvu de moyens de connexion auxiliaire 110 pour le raccordement simultanément sur ce même poste 10 d'autres types d'outils de décontamination, par exemple.

La figure 5 illustre l'utilisation du dispositif de décontamination 1 engagé dans un contenant 100 au travers d'une ouverture 108. Le fourreau rétractable 3, en position d'utilisation, prend appui aux abords de l'ouverture 108, sur le contenant 100 par l'intermédiaire de l'embase 5. La forme de l'embase 5 est configurée pour venir épouser la forme du contenant. Lors du retrait du dispositif de décontamination 1 en dehors du contenant 100, le fourreau rétractable 3 reste en appui au travers de l'embase 5 contre le contenant 100, autour de l'ouverture 108. La force de rappel du ressort 20 permet le coulissement du fourreau rétractable 3 le long du corps principal 2 jusqu'à ce que le fourreau rétractable 3 retrouve sa position de repos.

La lampe à rayonnement ultraviolet ou lampe UV-C 4 est de préférence une lampe à lumière pulsée. La forme de la lampe UV-C 4 est préférentiellement tubulaire et en forme de « U ».

Comme illustré sur les figures 2 et 3, la lampe UV-C 4 est fixée au niveau de sa première extrémité supérieure à la seconde extrémité inférieure du corps principal 2. Préférentiellement, à cette sa première extrémité supérieure de la lampe UV-C 4 celle-ci comporte une tête de lampe 41 par l'intermédiaire de laquelle elle est fixée au corps principal 2.

Par ailleurs, à cette première extrémité supérieure de la lampe UV-C, plus particulièrement à la tête de lampe 41, sont ménagés des éléments de maintien du dispositif de décontamination 1 dans le contenant à décontaminer. Plus particulièrement, les éléments de maintien sont formés par des pattes de maintien 411. Ces pattes de maintien 411 sont, de préférence, métalliques. Les éléments de maintien sont configurés pour se déployer une fois que le fourreau rétractable 3 a atteint sa fin de course. Ainsi, le contenant 100 est pris en tenaille entre l'embase 5 dont la forme épouse celle du contenant 100 et les éléments de maintien. Cette configuration permet ainsi de fixer le dispositif de décontamination 1 sur le contenant 100 et empêche son retrait accidentel. Le retrait du dispositif de décontamination 1 n'est permis que par l'application sur ce dernier d'une force de traction extérieure, par exemple exercée par un opérateur, force de traction supérieure à la force de retenu exercée par les pattes de maintien 411 sur le contenant 100.

Comme illustré sur la figure 2, le corps principal 2 comporte des moyens de préhension empruntant avantageusement la forme d'une poignée 210. Les moyens de préhension sont situés sur une première extrémité supérieure du corps principal 2. La première extrémité supérieure du corps principal 2 comprend préférentiellement une base 21 sur laquelle la poignée 210 est fixé. Avantageusement, les moyens de préhension comportent un revêtement qui en facilite la préhension par un opérateur.

A noter qu'au niveau de cette base 21 peuvent aboutir les moyens de raccordement d'alimentation 211. De plus, la base 21 du corps principal 2 comprend des moyens de positionnement du dispositif de décontamination 1, sur le poste d'alimentation et de pilotage 10 ou dans un contenant 100, en position verticale ou horizontale, afin de permettre une meilleure stabilisation du dispositif de décontamination 1. Plus particulièrement, les moyens de positionnement sont réalisés sous forme de crochets 6.

Comme illustré sur les figures 1 à 3, le fourreau rétractable 3 comprend des moyens d'évacuation d'air 33 configurés pour évacuer la chaleur dissipée par la lampe UV-C 4. Préférentiellement, les moyens d'évacuation d'air 33 sont formés par des orifices d'évacuation 34. Les moyens d'évacuation d'air 33 sont situés sur une partie supérieure de la portion principale 32 du fourreau rétractable 3. La partie supérieure de la portion principale 32 du fourreau rétractable 3 vient entourer la tête de lampe 41 dans la position de repos du fourreau rétractable 3. Cette configuration permet une meilleure évacuation de la chaleur lors d'une utilisation du dispositif de décontamination en position verticale.

Le dispositif de décontamination 1 comprend également des moyens de mesure du rayonnement émis par la lampe UV-C 4, préférentiellement constitués d'une photodiode, afin de permettre une mesure du niveau d'usure de la lampe UV-C 4 et de contrôler ainsi son efficacité. Préférentiellement, les moyens de mesure sont placés à l'intérieur du corps principal 2.

Le dispositif de décontamination 1 comporte un moyen de détection d'obscurité 60 tel que, par exemple, un capteur de luminosité. Tant que le capteur de luminosité détecte la lumière environnante, l'allumage de la lampe UV-C ne peut pas être enclenché.

L'allumage de la lampe UV-C 4 ne peut être enclenché que lorsque cette dernière est entièrement insérée dans le contenant 100 à décontaminer et se retrouve dans l'obscurité, ceci afin de permettre de protéger l'opérateur de la lumière émise par la lampe UV-C 4. Préférentiellement, le moyen de détection d'obscurité 60 est placé à l'extrémité inférieure du corps principal 2 à laquelle est fixée la lampe UV-C 4. Plus particulièrement il vient se situer à l'extrémité inférieure du corps principal 2, à proximité de la tête de lampe 41.

Comme illustré sur la figure 2, le dispositif de décontamination 1 comporte des moyens de détection de position du fourreau rétractable 3. Les moyens de détection peuvent, par exemple mais non limitativement, comprendre un capteur de position muni d'une première butée et d'une butée de fin de course ou capteur de position muni d'un contacteur à levier.

Selon un mode de réalisation, la butée de fin de course peut être située sur le corps principal 2 pour coopérer avec une première butée située à l'intérieur du fourreau rétractable 3 lorsque ce dernier atteint sa position d'utilisation. De manière avantageuse mais non limitative, la première butée peut être située sur la première extrémité supérieure du fourreau rétractable 3.

Selon un autre mode de réalisation, la butée de fin de course peut, par exemple, être située dans le fourreau rétractable 3 à proximité de l'embase 5, pour coopérer avec une première butée située sur la seconde extrémité inférieure du corps principal 2 lorsque le fourreau rétractable 3 atteint sa position d'utilisation.

Les moyens de détection de position du fourreau rétractable 3 sont configurés pour détecter la position de fin de course du fourreau rétractable 3 en position d'utilisation.

L'allumage de la lampe UV-C 4 ne peut être enclenché que lorsque les moyens de détection de positon détectent que le fourreau rétractable 3 a atteint la fin de sa course en position d'utilisation.

Le dispositif de décontamination 1 comprend également un dispositif d'allumage de la lampe UV-C 4. Ce dernier ne peut être activé que lorsque, la lampe UV-C est entièrement placée dans l'obscurité et que le fourreau rétractable 3 est arrivé en fin de course.

Avantageusement encore, le dispositif de décontamination comporte des moyens de transmission et d'alerte, notamment adaptés pour transmettre au poste d'alimentation et de pilotage 1 et/ou au dispositif d'allumage de ladite lampe UV-C (4) une information de vérification ou d'alerter, par exemple d'un disfonctionnement détecté par le moyen de détection d'obscurité et/ou les moyens de détection de positon du fourreau rétractable 3.

L'invention concerne également un procédé de mise en œuvre d'un dispositif de décontamination 1 conforme à l'invention et comprenant un poste d'alimentation et de pilotage 10 pour la décontamination d'un contenant alimentaire 100 pour la fabrication de vin ou d'autres boissons alcoolisées par rayonnement ultraviolet, préférentiellement par lumière pulsée.

Le procédé se caractérise en ce qu'il comporte :
- une étape d'application de l'embase 5 à la seconde extrémité du fourreau rétractable 3 au-dessus d'une l'ouverture 108 dans le contenant 100 ;
- une étape d'engagement de la lampe UV-C 4 à l'intérieur du contenant 100 au travers de l'ouverture 108 en repoussant le fourreau rétractable 3 depuis sa position de repos dans sa position d'utilisation, contre l'action des moyens de rappel élastique 2 ;
- une étape d'accrochage du dispositif de décontamination sur le contenant 100 ;
- une étape de vérification comprenant la vérification d'au moins deux conditions de sécurité parmi les suivantes :
   ∘ le moyen de détection d'obscurité détecte que la lampe UV-C 4 est placée dans l'obscurité ;
   ∘ les moyens de détection de positon du fourreau rétractable 3 détectent la fin de course de ce dernier qui vient en butée contre le contenant 100 ;
- une étape de transmission comprenant la transmission d'informations de vérification desdites conditions de sécurité à un poste d'alimentation et de pilotage 10 et/ou à un dispositif d'allumage de ladite lampe UV-C.
- une étape de validation dans laquelle :
   ∘ si au moins une desdites deux conditions de sécurité n'est pas vérifiée alors une alerte est émise audit poste d'alimentation et de pilotage 10 et/ou au dispositif d'allumage de ladite lampe UV-C (4) afin de contrôle et/ou réitération des étapes précédentes ;
   ∘ si toutes les conditions de sécurité sont vérifiées, ledit dispositif d'allumage de ladite lampe UV-C 4 peut alors être activé.

Lors de l'étape de positionnement du dispositif de décontamination 1 sur le contenant, l'embase 5 vient en appui contre le contenant, la forme de l'embase 5 vient épouser celle du contenant de sorte à éviter toute exposition d'un utilisateur aux fuites de rayonnement UV-C et un maintien en position du dispositif de décontamination 1 sur le contenant. Cette étape, peut comprendre l'utilisation de moyens de positionnement, réalisés de préférence sous forme de crochets 6, afin de permettre le positionnement et la stabilisation du dispositif dans le contenant 100.

Lors de l'étape d'accrochage du dispositif de décontamination 1, le fourreau rétractable 3 coulisse, depuis sa position de repos, le long du corps principal 2, jusqu'à sa position d'utilisation. Lorsque le fourreau rétractable arrive en fin de course, les pattes de maintien 411 se déploient sous le contenant. Le contenant est alors pris en tenaille entre l'embase 5 et les pattes de maintien 411 et permet ainsi le maintien en position et le blocage du dispositif de décontamination 1 dans le contenant 100.

## Revendications

1. Dispositif de décontamination d'un contenant alimentaire pour la fabrication de vin ou d'autres boissons alcoolisées par rayonnement ultraviolet, notamment de type lumière pulsée, comportant :
- Un corps principal (2) comportant des moyens de préhension fixés à une première extrémité supérieure dudit corps principal (2) ;
- Une lampe à rayonnement ultraviolet ou lampe UV-C (4) fixée à une seconde extrémité inférieure dudit corps principal (2) ;
- Un fourreau rétractable (3) monté coulissant sur ledit corps principal (2) entre une position de repos dans laquelle ladite lampe UV-C (4) est entièrement insérée dans ledit fourreau rétractable (3) et une position d'utilisation dans laquelle ladite lampe UV-C (4) s'étend en dehors dudit fourreau rétractable (3) à une seconde extrémité inférieure de ce dernier contre l'action de moyens de rappel élastique en position de repos ;
- Une embase (5) situé sur ladite seconde extrémité inférieure dudit fourreau rétractable (3), ledit embase (5) venant en appui contre ledit contenant (100) dans ladite position d'utilisation ;
**caractérisé en ce que** ledit dispositif (1) comporte :
- un moyen de détection d'obscurité (60) équipant le corps principal (2) à sa seconde extrémité inférieure à laquelle est fixée la lampe UV-C (4) ;
- des moyens de détection de position dudit fourreau rétractable (3) configurés pour détecter la position de fin de course du fourreau rétractable (3) en position d'utilisation ;
- un dispositif d'allumage de la lampe UV-C (4) conçu pour être activé que lorsque la lampe UV-C (4) est entièrement placée dans l'obscurité et que le fourreau rétractable (3) est arrivé en position de fin de course.

2. Dispositif de décontamination (1) selon la revendication 1, **caractérisé en ce qu'**il comporte un poste d'alimentation et de pilotage (10) auquel est raccordé le dispositif de décontamination (1) par des moyens de raccordement d'alimentation (211).

3. Dispositif de décontamination (1) selon la revendication 2, **caractérisé en ce que** le poste d'alimentation et de pilotage (10) comporte un pupitre de commande (102).

4. Dispositif de décontamination (1) selon l'une des revendications précédentes, **caractérisé en ce qu'**il comporte des moyens de transmission et d'alerte conçus pour transmettre une information de vérification et/ou d'alerter au poste d'alimentation et de pilotage (10).

5. Dispositif de décontamination (1) selon l'une quelconque des revendications précédentes, **caractérisé en ce qu'**il comporte des moyens de maintien dudit dispositif de décontamination (1) dans ledit contenant (100).

6. Dispositif de décontamination selon la revendication 5, **caractérisé en ce que** lesdits moyens de maintien sont situés au niveau d'une première extrémité supérieure de ladite lampe UV-C (4).

7. Dispositif de décontamination (1) selon la revendication 6, **caractérisé en ce que** ladite première extrémité supérieure de ladite lampe UV-C (4) comprend une tête de lampe (41) sur laquelle sont situés lesdits moyens de maintien.

8. Dispositif de décontamination (1) selon l'une des revendications 5 à 7, **caractérisé en ce que** lesdits moyens de maintien comportent au moins trois pattes de maintien (411).

9. Dispositif de décontamination selon l'une quelconque des revendications précédentes, caractérisé en ce lesdits moyens de préhension sont réalisés sous la forme d'une poignée (210).

10. Dispositif de décontamination selon l'une quelconque des revendications précédentes, **caractérisé en ce que** ledit fourreau rétractable (3) comporte des moyens d'évacuation d'air (33).

11. Dispositif de décontamination selon l'une quelconque des revendications 1 à 10, **caractérisé en ce que** le poste d'alimentation et de pilotage (10) est pourvu de moyens de connexion auxiliaire pour le raccordement d'autres types d'outils de décontamination.

12. Dispositif de décontamination selon l'une quelconque des revendications 1 à 11, **caractérisé en ce que** les moyens de détection de position comprennent un capteur de position du fourreau rétractable et une butée de fin de course ou encore un contacteur mécanique à levier.

13. Procédé de mise en oeuvre d'un dispositif de décontamination (1) selon les revendications 1 à 12 pour la décontamination d'un contenant (100) alimentaire pour la fabrication de vin ou d'autres boissons alcoolisées par rayonnement ultraviolet, notamment de type lumière pulsée, **caractérisé en ce qu'**il comporte :
- une étape d'application de l'embase (5) à la seconde extrémité du fourreau rétractable (3) au-dessus d'une ouverture (108) dans le contenant (100) ;
- une étape d'engagement de la lampe UV-C (4) à l'intérieur du contenant (100) en repoussant le fourreau rétractable (3) depuis sa position de repos dans sa position d'utilisation, contre l'action des moyens de rappel élastique (2) ;
- une étape d'accrochage du dispositif de décontamination (1) sur le contenant (100) ;
- une étape de vérification comprenant la vérification d'au moins deux conditions de sécurité parmi les suivantes :
o le moyen de détection d'obscurité détecte que la lampe UV-C (4) est placée dans l'obscurité ;
∘ le capteur de position du fourreau rétractable (3) détecte la fin de course de ce dernier qui vient en butée contre le contenant ;
- une étape de transmission comprenant la transmission à un poste de pilotage (10) et/ou à un dispositif d'allumage de ladite lampe UV-C (4) d'informations de vérification desdites conditions de sécurité ;
- une étape de validation dans laquelle :
∘ si au moins une desdites deux conditions de sécurité n'est pas vérifiée alors une alerte est émise audit poste d'alimentation et de pilotage 10 et/ou au dispositif d'allumage de ladite lampe UV-C (4) afin de contrôle et/ou réitération des étapes précédentes ;
o contenant (100) et que lesdites étapes précédentes soient réitérées ;
∘ si toutes les conditions de sécurité sont vérifiées, ledit dispositif d'allumage de ladite lampe UV-C 4 peut alors être activé.

## Patentansprüche

1. Vorrichtung zum Dekontaminieren eines Lebensmittelbehälters für die Herstellung von Wein oder anderen alkoholischen Getränken durch ultraviolette Strahlung, insbesondere von dem Typ Impulslicht, die aufweist:
- einen Hauptkörper (2), der Greifmittel aufweist, die an einem ersten oberen Ende des Hauptkörpers (2) befestigt sind;
- eine Ultraviolettstrahlungslampe oder UV-C-Lampe (4), die an einem zweiten unteren Ende des Hauptkörpers (2) befestigt ist;
- eine zurückziehbare Hülle (3), die auf dem Hauptkörper (2) zwischen einer Ruheposition, in der die UV-C-Lampe (4) vollständig in die zurückziehbare Hülle (3) eingeschoben ist, und einer Gebrauchsposition verschiebbar angebracht ist, in der sich die UV-C-Lampe (4) außerhalb der zurückziehbaren Hülle (3) an einem zweiten unteren Ende dieser letzteren gegen die Wirkung elastischer Rückstellmittel in der Ruheposition erstreckt;
- eine Basis (5), die sich an dem zweiten unteren Ende der zurückziehbaren Hülle (3) befindet, wobei die Basis (5) in der Gebrauchsposition an dem Behälter (100) anliegt;
**dadurch gekennzeichnet, dass** die Vorrichtung (1) aufweist:
- ein Dunkelheitserkennungsmittel (60), das den Hauptkörper (2) an seinem zweiten unteren Ende ausstattet, an dem die UV-C-Lampe (4) befestigt ist;
- Mittel zum Erkennen der Position der zurückziehbaren Hülle (3), die zum Erkennen der Endlageposition der zurückziehbaren Hülle (3) in der Gebrauchsposition konfiguriert sind;
- eine Vorrichtung zum Einschalten der UV-C-Lampe (4), die ausgelegt ist, um nur dann aktiviert zu werden, wenn die UV-C-Lampe (4) vollständig in der Dunkelheit platziert ist und die zurückziehbare Hülle (3) die Endlageposition erreicht hat.

2. Dekontaminationsvorrichtung (1) nach Anspruch 1,
**dadurch gekennzeichnet, dass** sie eine Stromversorgungs- und Steuerstation (10) aufweist, an die die Dekontaminationsvorrichtung (1) über Stromversorgungsanschlussmittel (211) angeschlossen ist.

3. Dekontaminationsvorrichtung (1) nach Anspruch 2,
**dadurch gekennzeichnet, dass** die Stromversorgungs- und Steuerstation (10) eine Schaltkonsole (102) aufweist.

4. Dekontaminationsvorrichtung (1) nach einem der vorstehenden Ansprüche, **dadurch gekennzeichnet, dass** sie Übertragungs- und Alarmmittel aufweist, die zum Übertragen von Überprüfungsinformationen und/oder Alarmieren der Stromversorgungs- und Steuerstation (10) ausgelegt sind.

5. Dekontaminationsvorrichtung (1) nach einem der vorstehenden Ansprüche, **dadurch gekennzeichnet, dass** sie Mittel zum Halten der Dekontaminationsvorrichtung (1) in dem Behälter (100) aufweist.

6. Dekontaminationsvorrichtung nach Anspruch 5,
**dadurch gekennzeichnet, dass** sich die Haltemittel an einem ersten oberen Ende der UV-C-Lampe (4) befinden.

7. Dekontaminationsvorrichtung (1) nach Anspruch 6,
**dadurch gekennzeichnet, dass** das erste obere Ende der UV-C-Lampe (4) einen Lampenkopf (41) aufweist, an dem sich die Haltemittel befinden.

8. Dekontaminationsvorrichtung (1) nach einem der Ansprüche 5 bis 7, **dadurch gekennzeichnet, dass** die Haltemittel mindestens drei Haltelaschen (411) aufweisen.

9. Dekontaminationsvorrichtung nach einem der vorstehenden Ansprüche, **dadurch gekennzeichnet, dass** die Greifmittel in Form eines Griffs (210) ausgeführt sind.

10. Dekontaminationsvorrichtung nach einem der vorstehenden Ansprüche, **dadurch gekennzeichnet, dass** die zurückziehbare Hülle (3) Abluftmittel (33) aufweist.

11. Dekontaminationsvorrichtung nach einem der Ansprüche 1 bis 10, **dadurch gekennzeichnet, dass** die Stromversorgungs- und Steuerstation (10) mit zusätzlichen Verbindungsmitteln für den Anschluss anderer Arten von Dekontaminationswerkzeugen versehen ist.

12. Dekontaminationsvorrichtung nach einem der Ansprüche 1 bis 11, **dadurch gekennzeichnet, dass** die Positionserkennungsmittel einen Positionssensor der zurückziehbaren Hülle und einen Endlageanschlag oder ein mechanisches Hebelschütz umfassen.

13. Verfahren zum Einsetzen einer Dekontaminationsvorrichtung (1) nach den Ansprüchen 1 bis 12 zum Dekontaminieren eines Lebensmittelbehälters (100) für die Herstellung von Wein oder anderen alkoholischen Getränken durch ultraviolette Strahlung, insbesondere von dem Typ Impulslicht,
**dadurch gekennzeichnet, dass** es aufweist:
- einen Schritt zum Anbringen der Basis (5) an dem zweiten Ende der zurückziehbaren Hülle (3) über einer Öffnung (108) in dem Behälter (100);
- einen Schritt zum Einsetzen der UV-C-Lampe (4) in den Behälter (100) durch Drücken der zurückziehbaren Hülle (3) aus ihrer Ruheposition in ihre Gebrauchsposition gegen die Wirkung der elastischen Rückstellmittel (2);
- einen Schritt zum Festmachen der Dekontaminationsvorrichtung (1) an dem Behälter (100);
- einen Überprüfungsschritt, umfassend die Überprüfung von mindestens zwei der folgenden Sicherheitsbedingungen:
∘ das Dunkelheitserkennungsmittel erkennt, dass die UV-C-Lampe (4) in der Dunkelheit platziert ist;
∘ der Positionssensor der zurückziehbaren Hülle (3) erkennt die Endlage dieser letzteren, die an dem Behälter in Anschlag kommt;
- einen Übertragungsschritt, umfassend die Übertragung von Informationen für die Überprüfung der Sicherheitsbedingungen an eine Steuerstation (10) und/oder an eine Vorrichtung zum Einschalten der UV-C-Lampe (4);
- einen Validierungsschritt, wobei:
o wenn mindestens eine der zwei Sicherheitsbedingungen nicht überprüft ist, eine Warnung an die Stromversorgungs- und Steuerstation 10 und/oder an die Vorrichtung zum Einschalten der UV-C-Lampe (4) ausgegeben wird, um die vorherigen Schritte zu kontrollieren und/oder zu wiederholen;
o Behälter (100) und dass die vorstehenden Schritte wiederholt werden;
o wenn alle Sicherheitsbedingungen überprüft wurden, die Vorrichtung zum Einschalten der UV-C-Lampe 4 aktiviert werden kann.

## Claims

1. Device for decontaminating a food container for the manufacture of wine or other alcoholic beverages by ultraviolet radiation, in particular of the pulsed light type, comprising:
- A main body (2) comprising gripping means attached to a first upper end of said main body (2);
- An ultraviolet lamp or UV-C lamp (4) attached to a second lower end of said main body (2);
- A retractable sleeve (3) slidably mounted on said main body (2) between a rest position in which said UV-C lamp (4) is fully inserted in said retractable sleeve (3) and a use position in which said UV-C lamp (4) extends out of said retractable sleeve (3) at a second lower end thereof against the action of elastic return means in the rest position;
- A base (5) located on said second lower end of said retractable sleeve (3), said base (5) bearing against said container (100) in said use position;
**characterized in that** said device (1) comprises:
- a darkness detection means (60) fitted to the main body (2) at its second lower end, to which the UV-C lamp (4) is attached;
- means for detecting the position of said retractable sleeve (3) configured to detect the end-of-travel position of the retractable sleeve (3) in the use position;
- a UV-C lamp (4) lighting device designed to be activated only when the UV-C lamp (4) is fully positioned in the dark and the retractable sleeve (3) has reached its end-of-travel position.

2. Decontamination device (1) according to claim 1, **characterized in that** it comprises a supply and control station (10) to which the decontamination device (1) is connected via supply connection means (211).

3. Decontamination device (1) according to claim 2, **characterized in that** the supply and control station (10) comprises a control console (102).

4. Decontamination device (1) according to one of the preceding claims, **characterized in that** it comprises transmission and warning means designed to transmit verification and/or warning information to the supply and control station (10).

5. Decontamination device (1) according to any of the preceding claims, **characterized in that** it comprises means for holding said decontamination device (1) in said container (100).

6. Decontamination device according to claim 5, **characterized in that** said holding means are located at a first upper end of said UV-C lamp (4).

7. Decontamination device (1) according to claim 6, **characterized in that** said first upper end of said UV-C lamp (4) comprises a lamp head (41) on which said holding means are located.

8. Decontamination device (1) according to one of claims 5 to 7, **characterized in that** said holding means comprise at least three holding tabs (411).

9. Decontamination device according to any of the preceding claims, **characterized in that** said gripping means take the form of a handle (210).

10. Decontamination device according to any of the preceding claims, **characterized in that** said retractable sleeve (3) comprises air evacuation means (33).

11. Decontamination device according to any of claims 1 to 10, **characterized in that** the supply and control station (10) is provided with auxiliary connection means for the connection of other types of decontamination tools.

12. Decontamination device according to any of claims 1 to 11, **characterized in that** the position detection means comprise a retractable sleeve position sensor and an end-of-travel stop or a mechanical lever contactor.

13. Method of operating a decontamination device (1) according to claims 1 to 12 for decontaminating a food container (100) for the manufacture of wine or other alcoholic beverages by ultraviolet radiation, in particular of the pulsed light type, **characterized in that** it comprises:
- a step of applying the base (5) at the second end of the retractable sleeve (3) over an opening (108) in the container (100);
- a step of engaging the UV-C lamp (4) inside the container (100) by pushing the retractable sleeve (3) from its rest position into its use position, against the action of the elastic return means (2);
- a step for attaching the decontamination device (1) to the container (100);
- a verification step comprising verification of at least two of the following safety conditions:
∘ the darkness detection means detects that the UV-C lamp (4) is placed in darkness;
∘ the position sensor of the retractable sleeve (3) detects the end of travel of said sleeve, which abuts against the container;
- a transmission step comprising the transmission to a control station (10) and/or to a lighting device of said UV-C lamp (4) of information verifying said safety conditions;
- a validation step in which:
o if at least one of said two safety conditions is not verified then an alert is issued to said supply and control station 10 and/or to the lighting device of said UV-C lamp (4) in order to check and/or repeat the preceding steps;
∘ container (100) and that said preceding steps are repeated;
o if all safety conditions are verified, said lighting device of said UV-C lamp 4 can then be activated.
